# EUROPEAN PATENT SPECIFICATION

(11) **EP 3 129 495 B1**
(45) Date of publication and mention of the grant of the patent: **23.08.2017**
(21) Application number: 15708952.5
(22) Date of filing: 13.02.2015
(51) Int. Cl.: C12Q 1/04, G01N 33/18, C12M 1/00

(54) **METHOD AND APPARATUS FOR DETERMINING MICROORGANISMS IN A WATER SAMPLE**
VERFAHREN UND GERÄT ZUR BESTIMMUNG MIKROORGANISMEN IN EINER WASSERPROBE
MÉTHODE ET APPAREIL DE DÉTERMINATION MICRO-ORGANISMES DANS UN ÉCHANTILLON D'EAU

(30) Priority: 09.04.2014 US 201461977330 P
(43) Date of publication of application: 15.02.2017
(73) Proprietor: Saudi Arabian Oil Company, Dhahran 31311 (SA); Danish Technological Institute, 8000 Aarhus C (DK)
(72) Inventor: AL-MONIEE, Mohammed, A., Dhahran 31311 (SA); TANG, Lone, DK-8000 Aarhus C (DK); JUHLER, Susanne, 2200 Kobenhavn N (DK); VOIGT, Niels, Vinther, 8200 Aarhus N (DK); SANDERS, Peter, Frank, DK-8000 Aarhus C (DK)
(74) Representative: Gervasi, Gemma
(86) International application number: PCT/US2015/015832
(87) International publication number: WO 2015/156906

(56) References cited:
- WO-A2-01/44500
- US-B2- 8 206 946
- P. ZHU ET AL: "Detection of water-borne E. coli O157 using the integrating waveguide biosensor", BIOSENSORS AND BIOELECTRONICS (ELSEVIER BV, NL), vol. 21, no. 4, 15 October 2005 (2005-10-15), pages 678-683, XP005098272, Amsterdam ISSN: 0956-5663, DOI: 10.1016/J.BIOS.2005.01.005

## Description

### FIELD OF THE INVENTION

The invention relates to methods and apparatus useful in continuous, automated, real time determination of microorganisms in flowing water systems, such as systems involving seawater, using a biosensor and DNA staining technology.

### BACKGROUND AND PRIOR ART

In any system involving flowing water, the presence of microorganisms in the water can influence the system in negative ways. Examples of such problems include microbially influenced corrosion of instruments, clogging of the system, or reservoirs, biofouling and so forth. See, e.g., U.S. Patent No. 8,525,130, incorporated by reference, which discusses problems caused by biofouling in seawater desalination plants, and efforts to detect and to analyze, e.g., biofilms which grow on the apparatus of the plants.

While there are many methodologies known for determining presence of microorganisms using biosensors, there is limited information available in the applicability of these methods to fluids, such as natural waters, recreational waters, seawater, and so forth. See in this regard, U.S. Patent No. 8,206,946, also incorporated by reference.

U.S. Patent No. 6,787,302, the disclosure of which is incorporated by reference, teaches the use of a commercially available dye "SYTO16," for determining viable cells in a fluid sample. Also see U.S. Patent No. 8,206,946, Published U.S. Application 20040191859, and PCT Application WO 1995 0191859, which disclose the use of fluorescent dyes for determining microorganisms. All are incorporated by reference.

None of these references teach or disclose methods and/or apparatus useful in real time analysis systems, which can be used for automated and continuous monitoring of the presence of microorganisms in water samples, such as seawater.

The invention which is disclosed infra is directed to apparatus and methods which address the issues set forth supra.

Further examples of apparatuses and/or methods for detecting microorganisms in water samples are disclosed in each of "P.ZHU ET AL: "Detection of water-borne E. coli 0157 using the integrating waveguide biosensor", BIOSENSORS AND BIOELECTRONICS (ELSEVIER BV, NL), vol. 21, no. 4, 15 October 2005 (2005-10-15), pages 678-683, XP005098272, Amsterdam ISSN: 0956:5663, DOI: 10.1016/J.BIOS.2005.01.005" and WO 01/44500.

### SUMMARY OF THE INVENTION

The invention relates to methods and apparatus useful in automated monitoring microorganisms in flowing water systems, such as seawater, on a continuous basis. Further, the invention comprises incorporating DNA staining technology into an autonomous microbe sensor, thus enabling detection of microorganisms via staining DNA, which is ubiquitous in microorganisms.

### BRIEF DESCRIPTION OF THE FIGURES

Figure 1 shows an embodiment of the invention.
Figure 2 shows results from one experiment carried out in accordance with the invention.
Figures 3a and 3b show further results.
Figure 4 shows yet further results.

### DETAILED DESCRIPTION OF PREFERRED EMBODIMENTS

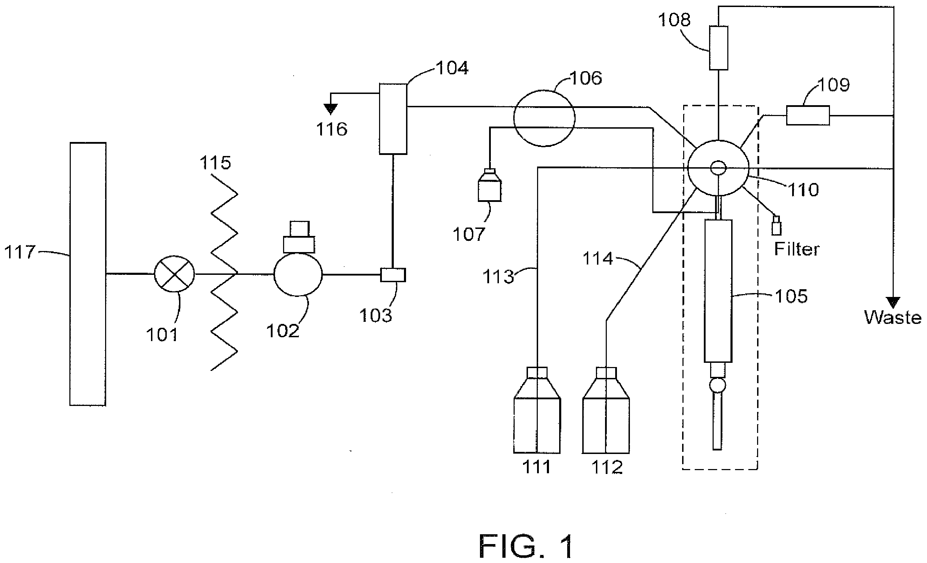

One embodiment of the invention is shown in Figure 1. Referring thereto a pipeline valve "101" is depicted, connected to pipeline 117. This serves as the connector interphase between the biosensor, discussed infra, and the seawater injecting side stream. Also depicted is a pressure regulator 102, which regulates the pressure of water injected into the biosensor, and ensures stable flow into a sampling chamber. As developed infra, the pressure of the water should be below 10 bar.

Delivery of the water in a stable, regular flow is also facilitated by a tubing means with a restrictor element "103." The water flows through the tubing means into sampling chamber "104." This chamber has a constant overflow, which ensures constant availability of fresh samples. Also shown is an automated syringe "105," which is calibrated to take precise sample volumes (e.g., 5 ml), as facilitated by a two port injection valve "106" and distribution valve "110". The injection valve 106 removes water from the sample chamber, and also from indicator reservoir "107." When sample and DNA stain are being mixed, automated syringe "105" draws the liquids back and forth, after which they move into mixing chamber "108," to further ensure uniform mixing. More generally, however, the syringe is used to take samples of, e.g., liquid from the sampling chamber, the indicator reservoir, reservoirs 111 and 112, discussed infra, and air from the air filter, not shown herein. Liquid or air can then be moved toward sampling chamber, indicator reservoir, the mixing chamber discussed infra, the flow cell, also discussed infra, and the water reservoir. Positions of valve 110 and injection valve 106 control the taking and distribution of sample. Also shown is flow cell "109," which may be viewed as a flow through cuvette, a first reservoir "111" containing, e.g., distilled water, and a second reservoir "112," which contains a cleaning agent, each of which are provided with means "113" and "114" for drawing the respective materials into the working apparatus are also shown. In a preferred embodiment, distribution valve 110 is motorized. As is shown in the embodiment of Figure 1, it has a plurality of ports (in this embodiment, 8, each of which is represented by a lead line), and it controls from where the syringe draws liquid or air, and to where the syringe content is distributed when the syringe is emptied. What is also shown herein is a portion of an enclosure means 115 used to protect the apparatus from the environment. The sensor itself is not shown in this figure. After measurement, of sample waste material is transferred via means 116 to, e.g., a waste reservoir, which is not shown.

In operation, the apparatus is turned to "on" mode automatically or manually. When this is done automatically, a timer, a computer control, inter or intranet connections, and so forth, may be used. When an automated timer is used, this is configured so as to turn on a computer using standard or customized software, or by programming a computer which is part of the biosensor, or by remote control, via interior intranet connection. Automated timer means utilize the least energy but other systems may be used.

As can be seen from the description supra, the apparatus of the invention uses a dispensing system to transport and mix fluids (e.g., water samples, dye solution, cleansing agents, and rinsing water). In the depicted embodiment, syringes are used, but the skilled artisan will see the possibility of other modes for dispensing and mixing.

In operation, a sample of water, e.g., seawater is removed from the sampling chamber, and a precise amount, as discussed infra, is mixed with an indicator, such as a DNA binding dye, in a predetermined, but variable ratio. The ratio depends on many factors including the nature of the indicator, the salinity of the liquid being tested, and other factors. In the examples, SYBR^{®} Green I ((N',N'-dimethyl-N-[4-[(E)-(3-methyl-1,3-benzothiazol-2-yildene)methyl]-1-phenylquinolin-1-um-2-yl]-N-propylpropane-1,3-diamine)) was mixed with seawater having 55% salinity at a 1:10,000 ratio. The mixture is incubated for a predetermined period of time, to allow penetration of the indicator through bacterial cell membranes and binding of indicator to, e.g., DNA. The choice of the length of incubation time will vary, and will increase depending upon the degree of sensitivity desired. In the examples which follow, the incubation time was 40 minutes.

After incubation, the sample is pumped to the flow cell, which has been adapted for detection of the indicator via, e.g., fluorescence, such as with an LED light source having an excitation wavelength of 490 nm. This parameter is used because the warm up time is short, and the energy demand is low. Entry and emission of light can be controlled via, e.g., optical filters to minimize interference, and emitted light is measured via spectrometry at 520 nm. It is well known that one can vary LED properties and filters depending upon the nature of the indicator used. Any data secured via the system described herein can be stored on a sensor computer, and is accessible directly or remotely. The skilled artisan will recognize that filters, and LED wavelengths can be changed depending upon the dye used.

The length of operation and number of measurements that may be taken in a given time period are dependent on factors such as the size of the reagent containers.

### EXAMPLE 1

The following example describes the use of the embodiment described supra. It permitted a set up run for 26 days, with 3 measurements a day before inspection and replacement of reagents were needed.

The biosensor was placed on a flat surface, and connected to a power supply (230 V AC, with live, neutral and earth for power supplies). It should be noted that the system can be adapted for use of solar panels, controls, and batteries.

Further, the sensor was connected to a side stream, having a pressure less than 10 bar. The connection is facilitated by a push in connector, which facilitates connection and replacement of the transport means, which brings water to the system.

In operation, the current system is designed to function at temperatures below 40°C. If temperatures rise above 40°C, incorporated components shut down the system. If components which are not temperature sensitive are used, or cooling means are incorporated into the system, this feature is unnecessary.

The following details the general manner in which the system works; however, variations are possible as will be recognized by the skilled artisan.

The biosensor first measures temperature and humidity to determine if limiting parameters (e.g., temperatures above 40°C, or humidity above 90%) are present. As noted supra, the equipment shuts down if this is the case.

The sensor also provides information on whether any reagents require replenishing.

If the environmental conditions are satisfactory and the necessary quantities of reagent are present, the spectrometer is warmed up, and the system is rinsed with a sample. Following this, both a water sample and a quantity of dye solution using pre-set ratios, are drawn into the syringe. These are mixed via pumping liquids back and forth to the mixing chamber, e.g., three times. After mixing, the sample is incubated, dosed to the flow cell, and fluorescence detected.

Following this, the system is flushed with cleaning agent, distilled water, air, and then distilled water again. The system is then shut down, with automated instructions inputted as to when the process should repeat.

The invention as described herein reduces the time necessary to analyze water samples from weeks, to hours.

### EXAMPLE 2

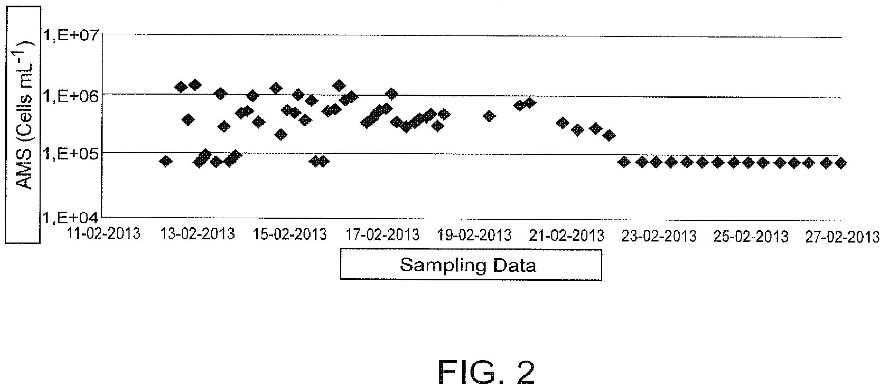

Water supplies were analyzed using the methodology set forth supra. It can be seen, from Figure 2, that microbial content was high *ab initio*; however, at the point indicated by the arrow (ten days after measurements begun), a biocide was added, and values were below detection limits. (Note that the data of Figure 2 represents a correlation between fluorescence signals, and manual counting).

### EXAMPLE 3

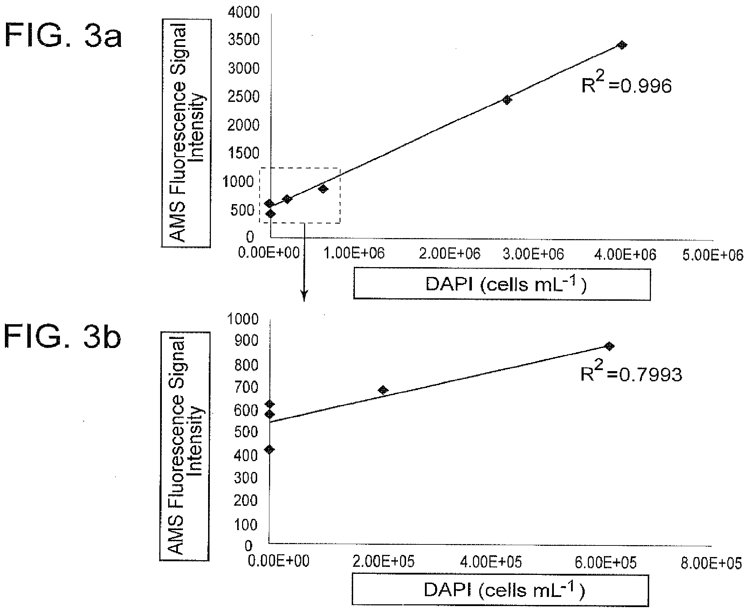

Long-term field-testing of the invention described herein was carried out in Saudi Arabia. Figures 3a and 3b present the data, again in terms of correlation of fluorescent staining and manual counting. The linear correlation allows for conversion to cell numbers (cells/ml of seawater). Conversion factors will differ for every system based upon *inter alia* the chemical composition of the sample, the dye and microbe size.

### EXAMPLE 4

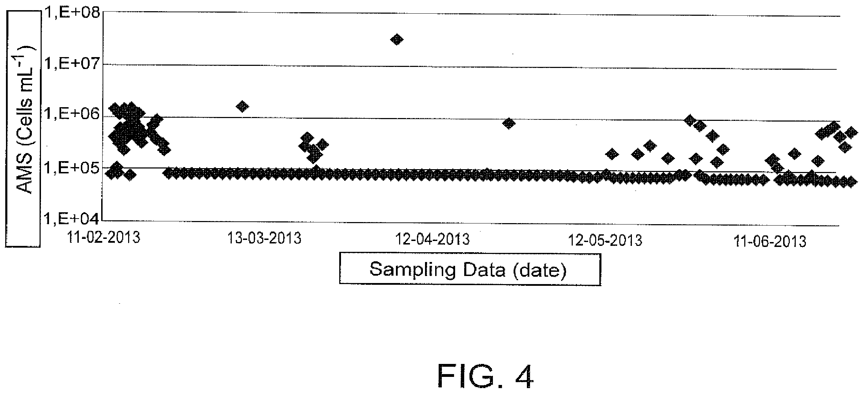

In long-term field tests, microbial content of seawater was measured three times a day, over a 4-month period. The results, shown in Figure 4, provided valuable information not only about microbial presence but periods where growth rates increased, or decreased. For example, following biocide treatment, there were no detectable microbes, after which growth rates increased.

Other embodiments will be clear to the skilled artisan and need not be reiterated here.

The terms and expression which have been employed are used as terms of description and not of limitation; the present invention is defined by the claims.

## Claims

1. Apparatus useful in the continuous, automated, real time determination of the presence of microorganisms in a water sample comprising a biosensor connected via a first valve means (101) to a means (117) for providing a water sample to said biosensor, said biosensor further comprising a pressure regulator means (102) and a restrictor means (103) which regulate flow of water to a sample chamber (104), said sample chamber (104) having a first transport means for transport of a volume of sample to a mixing chamber (108), an indicator reservoir (107) having a second means for transport of indicator which is a DNA stain to said mixing chamber (104), wherein said first and second means for transport have connected thereto an injection valve means (106) for controlling volume of sample and indicator; said mixing chamber (108) having a syringe means (105) connected thereto, said syringe means (105) operating to mix said sample and indicator, means for containing an analysis sample in fluid connection with said mixing chamber (108), and a means for determining said indicator in said means for containing said analysis sample.

2. The apparatus of claim 1, wherein said means for determining said indicator is a spectrofluorometer.

3. The apparatus of claim 1, further comprising a first reservoir means (111) connected to said biosensor for delivery a quantity of a cleansing agent.

4. The apparatus of claim 3, further comprising a second reservoir means (112) for delivering cleaning water to said biosensor.

5. The apparatus of claim 1, further comprising a waste disposal means connected to said mixing chamber (108).

6. A method for continuous, automated, real time determination of presence of microorganisms in a liquid sample comprising transporting a liquid sample to the mixing chamber (108) of the apparatus of claim 1 together with an DNA stain indicator sample to form a mixture, incubating said mixture, and determining uptake of said DNA stain indicator has taken place as an indicator of presence of microorganisms.

7. The method of claim 6, wherein said liquid sample is seawater.

8. The method of claim 6, wherein said indicator is SYBR^{®} green I.

## Patentansprüche

1. Vorrichtung geeignet für die kontinuierliche, automatisierte Echtzeitdetermination von Mikroorganismen in einer Wasserprobe, aufweisend einen Biosensor, der über erste Ventileinrichtungen (101) mit einer Vorrichtung (117) verbunden ist, um dem Biosensor eine Wasserprobe bereitzustellen, wobei der Biosensor zudem eine Druckregelung (102) und eine Drosselung (103) aufweist, die den Wasserdurchfluss zu einer Probenkammer (104) regulieren, wobei die Probenkammer (104) ein erstes Transportmittel für den Transport eines Probenvolumens zu einer Mischkammer (108) aufweist, und ein Indikatorreservoir (107) ein zweites Transportmittel für den Transport des Indikators, der ein DNA-Farbstoff ist, zu der Mischkammer (104) aufweist, wobei das erste und zweite Transportmittel daran angebundene Einspritzventile (106) zur Kontrolle der Volumina der Probe und des Indikator haben; wobei
die Mischkammer (108) eine mit ihr verbundene Einspritzeinrichtung (105), wobei die Einspritzeinrichtung (105) betrieben wird, um die Probe und den Indikator zu mischen, und eine Einrichtung zur Aufnahme einer Analysenprobe, die in Flüssigkeitsverbindung mit der Mischkammer (108) steht, sowie eine Einrichtung zur Determination des Indikators in der Einrichtung zur Aufnahme der Analysenprobe aufweist.

2. Vorrichtung gemäß Anspruch 1, wobei die Einrichtung zur Determination des Indikators ein Spektrofluorometer ist.

3. Vorrichtung gemäß Anspruch 1, zudem aufweisend ein erstes Reservoir (111), das mit dem Biosensor zur Zuführung einer Menge an Reinigungsmittel verbunden ist.

4. Vorrichtung gemäß Anspruch 3, zudem aufweisend ein zweites Reservoir (112) zur Zuführung von Reinigungswasser zu dem Biosensor.

5. Vorrichtung gemäß Anspruch 1, zudem aufweisend eine Abfallentsorgung, die mit der Mischkammer (108) verbunden ist.

6. Verfahren zur kontinuierlichen, automatisierten Echtzeitdetermination von Mikroorganismen in einer Flüssigkeitsprobe, aufweisend den Transport einer Flüssigkeitsprobe zusammen mit einer DNA-Farbstoff-Indikatorprobe zur Mischkammer (108) der Vorrichtung gemäß Anspruch1, um eine Mischung herzustellen, die Mischung zu inkubieren und die Aufnahme des DNA-Farbstoff-Indikators zu bestimmen, die als Indikator für Mikroorganismen dient.

7. Verfahren gemäß Anspruch 6, wobei die Flüssigkeitsprobe Meerwasser ist.

8. Verfahren gemäß Anspruch 6, wobei der Indikator SYBR® green I ist.

## Revendications

1. Appareil utile dans la détermination en temps réel automatisée et continue de la présence de micro-organismes dans un échantillon d'eau comprenant un biocapteur raccordé via un premier moyen de vanne (101) à un moyen (117) de fourniture d'un échantillon d'eau audit biocapteur, ledit biocapteur comprenant en outre un moyen régulateur de pression (102) et un moyen d'étranglement (103) qui régulent l'écoulement d'eau vers une chambre à échantillon (104), ladite chambre à échantillon (104) ayant un premier moyen de transport pour le transport d'un volume d'échantillon vers une chambre de mélange (108), un réservoir indicateur (107) ayant un second moyen de transport d'indicateur qui est un colorant à ADN vers ladite chambre de mélange (104), dans lequel lesdits premier et second moyens de transport sont raccordés à un moyen de vanne d'injection (106) permettant de contrôler le volume d'échantillon et d'indicateur ; ladite chambre de mélange (108) ayant un moyen de seringue (105) raccordé, ledit moyen de seringue (105) fonctionnant pour mélanger lesdits échantillon et indicateur, un moyen destiné à contenir un échantillon d'analyse en raccordement fluidique avec ladite chambre de mélange (108), et un moyen de détermination dudit indicateur dans ledit moyen destiné à contenir ledit échantillon d'analyse.

2. Appareil selon la revendication 1, dans lequel ledit moyen de détermination dudit indicateur est un spectrofluoromètre.

3. Appareil selon la revendication 1, comprenant en outre un premier moyen de réservoir (111) raccordé audit biocapteur pour livraison d'une quantité d'un agent de nettoyage.

4. Appareil selon la revendication 3, comprenant en outre un second moyen de réservoir (112) pour livrer de l'eau de nettoyage audit biocapteur.

5. Appareil selon la revendication 1, comprenant en outre un moyen d'élimination de déchet raccordé à ladite chambre de mélange (108).

6. Procédé de détermination en temps réel automatisée et continue de la présence de micro-organismes dans un échantillon liquide comprenant le transport d'un échantillon liquide vers la chambre de mélange (108) de l'appareil de la revendication 1 conjointement avec un échantillon indicateur à colorant à ADN pour former un mélange, l'incubation dudit mélange, et la détermination qu'une capture dudit indicateur à colorant à ADN a eu lieu en tant qu'indicateur de la présence de micro-organismes.

7. Procédé selon la revendication 6, dans lequel ledit échantillon liquide est de l'eau de mer.

8. Procédé selon la revendication 6, dans lequel ledit indicateur est SYBR^{®} green I.
